# EUROPEAN PATENT APPLICATION

(11) **EP 3 185 188 A1**
(43) Date of publication of application: **28.06.2017**
(21) Application number: 15202196.0
(22) Date of filing: 22.12.2015
(51) Int. Cl.: G06Q 10/06, G06Q 50/22

(54) **TASK MANAGEMENT SYSTEM AND METHOD FOR ASSIGNING TASKS IN A TASK MANAGEMENT SYSTEM**

(71) Applicant: Televic Healthcare NV, 8870 Izegem (BE)
(72) Inventor: Dereuddre, Wim, 8820 Torhout (BE); Bhatti, Jabran, 8400 Oostende (BE); Crombez, Pieter, 8820 Torhout (BE); Verborgh, Ruben, 9050 Gentbrugge (BE); Dörthe, Arndt, 9000 Gent (BE); De Meester, Ben, 9260 Schellebelle (BE)
(74) Representative: Beck, Michaël Andries T.

(57) **Abstract**

The invention pertains to a method for assigning tasks, comprising: receiving a task request (100); retrieving prioritization criteria (130) and ranking values and justification texts for the prioritization criteria; applying (140) a subset of the criteria to the staff profiles, and associating the ranking value and justification text with the staff profiles for the prioritization criteria fulfilled by the staff profiles; selecting (150) a staff profile from among the plurality of staff profiles having the highest ranking value associated with it; and outputting (160) an assignment message comprising information indicative of the task request, the selected staff profile, and the justification text associated with the highest ranking value; wherein the subset is determined so as to exclude prioritization criteria the negative outcome of which can be deduced by applying rules of formal logic to the outcome of other prioritization criteria within the subset.

## Description

### Field of the Invention

The present invention pertains to automated task management systems, in particular systems designed to allocate incoming task requests in real-time to staff members on the basis of predetermined criteria.

### Background

Task management systems are known and are typically used in organizations such as hospitals, to allocate appropriately qualified care givers to patients when they request assistance.

It is an inconvenience of the known systems that the staff member to whom a given task is allocated, does not know on the basis of which criterion he or she has been selected instead of any other staff member.

It is an object of embodiments of the present invention to overcome this inconvenience.

### Summary of the Invention

According to an aspect of the present invention, there is provided a method for assigning tasks in an automated task management system, the task management system comprising a plurality of staff profiles, the staff profiles comprising static and regularly updated dynamic properties of staff members, the method comprising: receiving a task request; retrieving a set of prioritization criteria and respective associated ranking values and justification texts for the prioritization criteria; applying a subset of the set of prioritization criteria to the plurality of staff profiles, and associating the associated ranking value and the associated justification text with each one of the staff profiles for each prioritization criterion of the minimal subset that is fulfilled by the one of the staff profiles; selecting for the task request a staff profile from among the plurality of staff profiles having the highest ranking value associated with it; and outputting an assignment message comprising information indicative of the task request, information indicative of the staff profile selected for the task request, and the justification text associated with the highest ranking value; wherein the subset of the set of prioritization criteria is determined so as to exclude prioritization criteria the negative outcome of which can be deduced by applying rules of formal logic to the outcome of other prioritization criteria within the subset.

The present invention is based *inter alia* on the insight of the inventors that by storing information (rankings and justifications) about the applied prioritization criteria as they are being applied, the ability to immediately provide a full justification of the resulting selection is obtained. The present invention is further based on the insight that the selection process can be made more efficient by taking into account logical interdependencies between prioritization criteria, an efficiency gain which immediately applies to the process of generating a justification as well, since prioritization criteria that need not be applied do not give rise to the storage of justification texts either.

In an embodiment of the method according to the present invention, the task request comprises at least one task attribute, and the retrieving of the set of prioritization criteria comprises associating prioritization criteria with the at least one task attribute.

It is an advantage of this embodiment that the system can be used where the tasks are non-homogenous, i.e. when different types of tasks must be assigned according to different criteria. In a hospital context, a task may for example be classified as "paramedical" or "comfort", resulting in an exemplary prioritization of nurses and otherwise qualified caregivers, respectively.

In an embodiment, the method according to the present invention further comprises: retrieving a set of filter criteria and respective associated ranking values and justification texts for said filter criteria; applying a subset of said set of filter criteria to said plurality of staff profiles, and associating said associated ranking value and said associated justification text with each one of said staff profiles for each filter criterion of said subset that is fulfilled by said one of said staff profiles; and the subset of the set of prioritization criteria is only applied to those of the plurality of staff profiles that fulfill all criteria of the subset of filter criteria.

It is an advantage of this embodiment that a binary selection can be carried out, prior to running the computationally intensive prioritization rules. This results in a further gain of efficiency, in cases where certain staff members can be excluded a *priori* from being assigned the task on the basis of a filter criterion. To avoid getting into a situation where the task is assigned to nobody, a fallback scenario is provided where the individual filter criteria are ignored if they would disqualify all staff members. As the selected staff member, in this hypothesis, would normally not be eligible for the task according to one of the filter criteria, it is recommended in this case to inform the selected staff member that the selection has taken place by ignoring the filtering rules, so as to allow the selected staff member to seek additional assistance or advice for carrying out the task. To this end, the non-application of the filter criteria may be recorded in an additional justification text.

In a particular embodiment, the task request comprises at least one task attribute, and the retrieving of the set of filter criteria comprises associating filter criteria with the at least one task attribute.

It is an advantage of this embodiment that the system can be used where the tasks are non-homogenous, i.e. when different types of tasks must be assigned according to different criteria.

In an embodiment of the method according to the present invention, the applying is carried out by means of a rule-based reasoning engine.

It is an advantage of this embodiment that the selection can be performed very efficiently, as the rule-based reasoning engine is particularly well suited to apply sets of rules in which the outcome of some rules may affect the applicability of other rules.

According to an aspect of the present invention, there is provided a computer program product comprising code means configured to cause a processor to carry out the method as described above.

According to an aspect of the present invention, there is provided a task request system comprising a task management system configured to carry out the method as described above, a plurality of call terminals, and a plurality of paging terminals; wherein the call terminals are operatively connected to the task management system to convey a task request to the task management system; and wherein the paging terminals are operatively connected to the task management system to receive and display the assignment message.

The technical effects and advantages of the embodiments of the computer program product and the task request system according to the present invention correspond *mutatis mutandis* to those of the corresponding embodiments of the method according to the present invention.

### Brief Description of the Figures

These and other technical effects and advantages of embodiments of the present invention will now be further described with reference to the accompanying drawings, in which:
- Figure 1 provides a flow chart of a method according to an embodiment of the present invention;
- Figure 2 provides diagrams of a system according to an embodiment of the present invention; and
- Figure 3 illustrates an exemplary decision tree for use in a method according to an embodiment of the present invention.

### Description of Embodiments

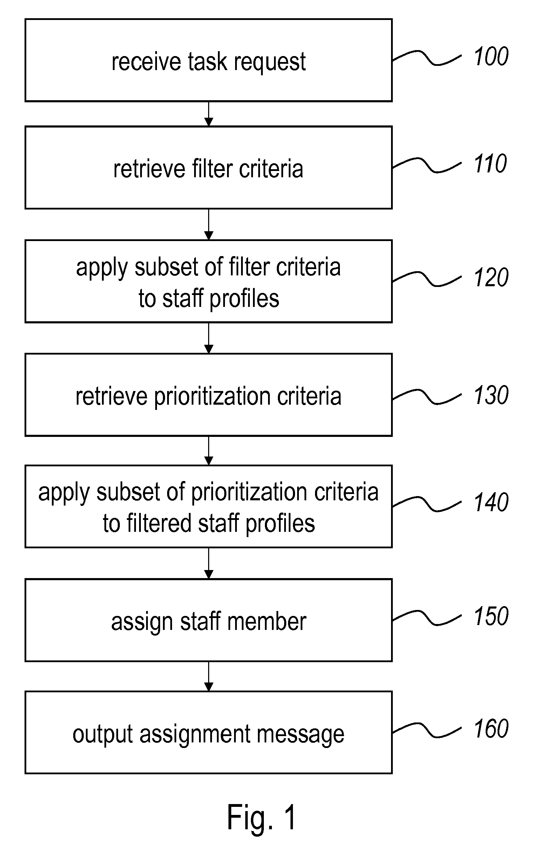

Figure 1 provides a flow chart of a method for assigning tasks in an automated task management system according to an embodiment of the present invention. The automated task management system comprises a plurality of staff profiles, which comprise static and regularly updated dynamic properties of staff members. The static properties of staff members don't vary over the time scales relevant for the application of the task assignment method; these include their qualifications, their long-term schedule, and the like. The dynamic properties of staff members do vary; these include whether they are presently occupied, their location in the building or complex, and the like. Thus, the selection of an appropriate staff member for a given task may vary depending on the time at which the task request is received, in view of the changing dynamic properties of the staff members.

The method is started with the receipt of a task request **100** via an appropriate interface. The task request may include task attributes that affect the filtering and/or prioritization criteria that should be applied to assign this task.

Optionally, a set of filter criteria is retrieved **110** along with respective associated ranking values and justification texts for these filter criteria. The filter criteria may be applied sequentially, leading to a gradual reduction of the available candidates for the task. To avoid ending up in a situation where none of the staff members is selected, individual filter criteria that would exclude all of the remaining staff members are skipped...

A set of prioritization criteria is retrieved **130** along with respective associated ranking values and justification texts for these prioritization criteria. A minimal subset of these prioritization criteria is applied **140** to the staff profiles, and the associated ranking value and justification text is associated with each one of the staff profiles for each prioritization criterion of the subset that is fulfilled by the staff profiles. The minimal subset of the set of prioritization criteria is determined so as to exclude prioritization criteria the negative outcome of which can be deduced by applying rules of formal logic to the outcome of other prioritization criteria within the subset.

After filtering and prioritization, a staff profile is selected **150** for the task request from among the staff profiles that have the highest ranking value associated with it. An assignment message comprising information indicative of the task request, information indicative of the staff profile selected for the task request, and the justification text associated with the highest ranking value is then output **160.**

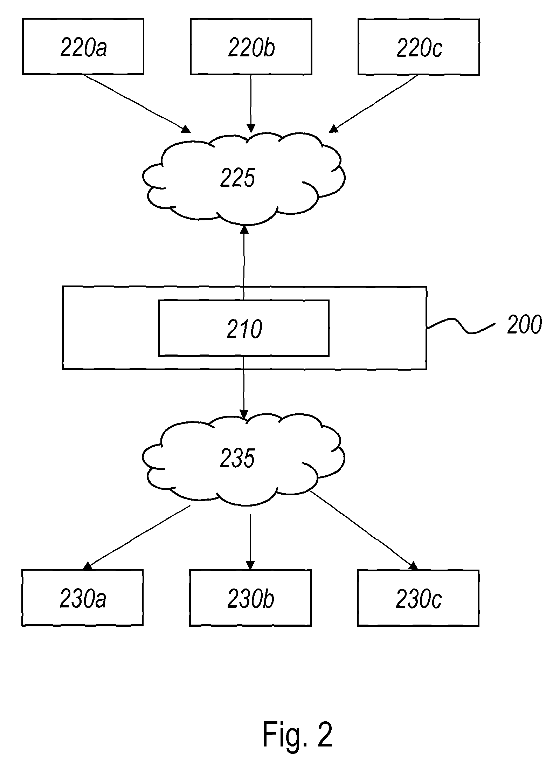

Figure 2 provides diagrams of a task request system **200** according to an embodiment of the present invention. The illustrated task request system **200** comprises a task management system **210,** a plurality of call terminals **220a-c,** and a plurality of paging terminals **230a-c.** Without loss of generality, only three call terminals are shown in Figure 2, and only three paging terminals are shown in Figure 2.

The call terminals **220a-c** are operatively connected to said task management system **210** to convey task requests to the task management system **210.** To this end, they are connected to an appropriate network infrastructure **225,** e.g. a wired network infrastructure such as an IEEE 802.3 "Ethernet" network or a wireless network infrastructure such as an IEEE 802.11 "Wi-Fi" network. The call terminals **220a-c** may be "nurse call terminals" of the type used in hospitals and other care institutions.

The paging terminals **230a-c** are operatively connected to the task management system **210** to receive and display said assignment message. To this end, they are connected to an appropriate network infrastructure **235,** typically a wireless network infrastructure such as a DECT network, a mobile telephony and data network, or an IEEE 802.11 "Wi-Fi" network. The paging terminals **230a-c** may be pagers or mobile devices of the type used in hospitals and other care institutions.

The task request system **200** is configured to carry out the method according to the invention; thus, all optional features and details described above with reference to the method according to the invention apply equally to the task request system. The functions of the task request system **200** may be implemented in dedicated hardware (e.g., ASIC), configurable hardware (e.g., FPGA), programmable components (e.g., a DSP or general purpose processor with appropriate software), or any combination thereof. The same component(s) may also include other functions.

The present invention also pertains to a computer program product comprising code means configured to cause a processor to carry out the method as described above. The computer program product may consist of the code means as provided on a computer-readable medium, including but not limited to magnetic, optical, or solid-state storage media.

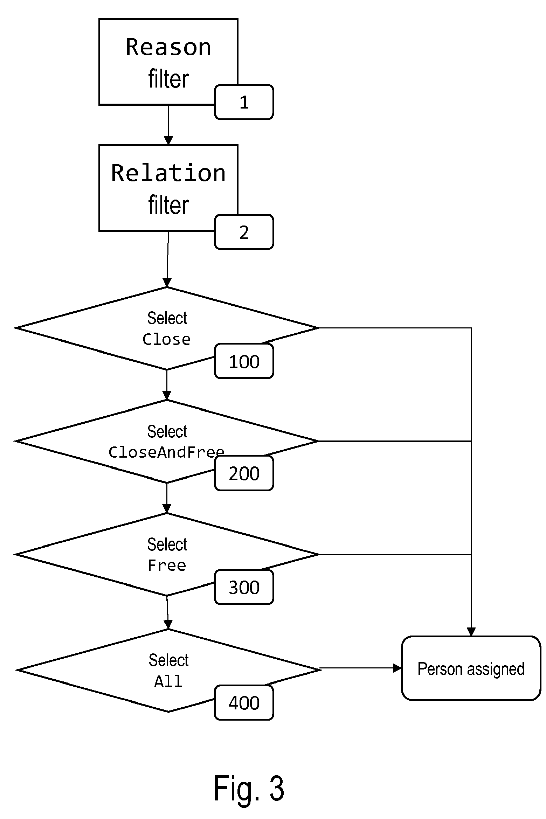

A use case of a method according to an embodiment of the present invention will now be described with reference to the exemplary decision tree illustrated in Figure 3.

As a new task request (a "call") comes in, all available staff is initially selected, i.e. a first filter criterion (not shown) is the "available" status of the staff members.

The available staff is filtered to those people who can handle the request **(Reason** filter; e.g., a task having the "MedicalCall" attribute can only be solved by a staff member having the "Doctor" property). The **Reason** filter is a filter criterion with an exemplary ranking value of "1", and an exemplary justification might be: "You have been selected because you are a doctor and the call is of a medical nature". At this stage, if nobody is competent according to this filter criterion, the filter is skipped, and everyone is returned.

The filtered staff is again filtered to those people who have a good relation with the patient **(Relation** filter); this filtering step requires that the profiles of the staff members include a dynamic property assessing the quality of their relationship with individual patients. The **Relation** filter is a filter criterion with an exemplary ranking value of "2", and an exemplary justification might be: "You have been selected because you have a good relationship with this patient". Again, at this stage, if nobody has the desired relationship value according to this filter criterion, the presently selected set of staff members is returned in its entirety.

Subsequently, the prioritization criteria (or "selectors") are applied:
- **CloseAndFree** selector:
   ∘ The staff that is close and free is selected
   ∘ The selection is associated with a certain ranking value, e.g. "100"
   ∘ The selection is associated with a certain justification text, e.g. "you have been selected because you are near the source of the call and presently not otherwise occupied"
- **Free** selector:
   o The staff that is free is selected
   o The selection is associated with a certain ranking value, e.g. "200"
   o The selection is associated with a certain justification text, e.g. "you have been selected because you are presently not otherwise occupied"
- **Close** selector:
   ∘ The staff that is nearby is selected
   ∘ The selection is associated with a certain ranking value, e.g. "300"
   ∘ The selection is associated with a certain justification text, e.g. "you have been selected because you are near the source of the call"

The justification text may contain dynamic information. For example, in the case of the **Close** selector, the actual location of the call and the staff member may be mentioned. The location of the call may be obtained from the call terminal. The location of the staff member is a dynamic property that must be regularly updated by the task management system; to this end, the task management system may receive information from a localization system, which may be based on tags worn by the staff members, which interact with beacons present throughout the facility.

Without loss of generality, the indicated ranking values are assigned in such a way that a lower value indicates a higher preference. While these rules are shown in a particular order in Figure 3, this is not necessarily the order in which they are applied, especially for the priority rules (selectors). Note for example that if a particular staff member yields a negative result for the **CloseAndFree** selector **(CloseAndFree** == FALSE) and a positive result for the **Free** selector **(Free** == TRUE), it is no longer necessary to test the **Close** selector, as it is logically derivable that it must be negative **(Close** == FALSE).

One or more staff members are assigned to solve the incoming call, on the basis of the ranking value of the selectors for which they were a positive match.

In an exemplary hospital situation, there is a patient Eric, and the staff consists of nurses Carl, Lisa, and Josephine, and doctor Ellen. Patient Eric prefers to be treated by Carl, Lisa, or Ellen **(TrustRelationShip).**

Their physical distribution in the hospital is as follows:

| Hallway | | |
|---|---|---|
| Patient Room {Eric} | Patient Room {Carl, Josephine} | Patient Room {Lisa, Ellen} |

In this physical layout, Carl and Josephine are deemed to be "close" to patient Eric, because they are in the next room, while Lisa and Ellen are not deemed to be "close" to patient Eric. All staff members are assumed to be "free", e.g. not presently otherwise occupied.

In a first exemplary call, Eric calls for help because he fears he is having a heart attack, or cardiological monitoring equipment places the call on his behalf. This call has the attribute **"MedicalCall".** The following selection steps would take place:
- {Carl, Lisa, Josephine, Ellen} → **Reason** filter → {Ellen(1)}
- {Ellen} → **Relation** filter → {Ellen(2)}
- {Ellen} → **Free** selector → {Ellen(200)}

Note that after the application of the **Reason** filter and the **Relation** filter, none of the remaining staff members is close, so the **Close** selector rule does not lead to any ranking value assignments, and given this result, the **CloseAndFree** selector rule need not be applied. In this case, Ellen is assigned to this call. The determining criterion is the **Reason** filter, which resulted in the selection of a single remaining staff member, with a ranking value of 1, and which will yield the justification text: "You have been selected because you are a doctor and the call is of a medical nature". Justification texts related to the application of selector rules may be added; this would be particularly useful if the filter stage had yielded more than one candidate.

In a second exemplary call, Eric wants a glass of water. This call has the attribute **"ComfortCall".** The following selection steps would take place:
- {Carl, Lisa, Josephine, Ellen} → **Reason** filter → {Carl(1), Lisa(1), Josephine(1), Ellen(1)}
- {Carl, Lisa, Josephine, Ellen} → **Relation** filter → {Carl(2), Lisa(2), Ellen(2)}
- {Carl, Lisa, Ellen} → **CloseAndFree** selector → {Carl(100)}
- {Carl, Lisa, Ellen} → **Free** selector → {Carl(200), Lisa(200), Ellen(200)}

Hence, Carl is assigned to this call. The determining prioritization criterion is the **CloseAndFree** selector, which resulted in a ranking value of 100 (better than the ranking value of 200 for the **Free** selector), and which will yield the justification text: "you have been selected because you are near the source of the call and presently not otherwise occupied" (in addition to justification texts related to the application of the **Reason** filter and the **Relation** filter, if desired).

In a third exemplary call, Eric wants a glass of water, but Carl is busy. This call has the attribute "ComfortCall". The following selection steps would take place:
- {Carl, Lisa, Josephine, Ellen} → **Reason** filter → {Carl(1), Lisa(1), Josephine(1), Ellen(1)}
- {Carl, Lisa, Josephine, Ellen} → **Relation** filter → {Carl(2), Lisa(2), Ellen(2)}
- {Carl, Lisa, Ellen} → **Free** selector → {Lisa(200), Ellen(200)} Note that after the application of the **Reason** filter, the **Relation** filter, and the **Free** selector, none of the remaining staff members is close, so the **Close** selector rule does not lead to any ranking value assignments, and given this result, the **CloseAndFree** selector rule need not be applied. In this case, Lisa or Ellen is assigned to this call. The determining prioritization criterion is the **Free** selector, which resulted in the selection of two remaining staff members with a ranking value of 200, and which will yield the justification text: "you have been selected because you are presently not otherwise occupied" (in addition to justification texts related to the application of the **Reason** filter and the **Relation** filter, if desired).

Every filter criterion is a set of rules that has, as input, the remaining staff members and outputs the filtered staff members. The sequence of these filters can be configured.

Every prioritization criterion (selector rule) is one rule that outputs the conforming staff members. The order of these selector rules can also be configured. If multiple selector rules are triggered, the output of the top selector rule is used as output (e.g., selector **CloseAndFree** will be a subset of selector **Close,** but the selector **CloseAndFree** is more important, so this output will be used).

These rules may be fed to a rule-based reasoning engine, such as the EYE reasoner ("Euler Yet another proof Engine", see http://eulersharp.sourceforge.net/), together with initial data, and possible extra rule files to allow for more complex reasoning.

When requesting the most appropriate staff member to be assigned to the task, all data and rule files are fed to an instance of the reasoning engine, which reasons over everything as a single process, and when the reasoning engine finishes, the output (i.e., the staff member to be assigned) is returned.

To show *why* a certain person is assigned to a certain call, the operation of the reasoning engine is advantageously leveraged. The reasoner will only trigger the rules that can be triggered, e.g., if no staff member is free, the **CloseAndFree** selector and **Free** selector will not trigger.

While the invention has been described hereinabove with reference to specific embodiments, this was done to clarify and not to limit the invention. The skilled person will appreciate that various modifications and different combinations of disclosed features are possible without departing from the scope of the invention.

## Claims

1. A method for assigning tasks in an automated task management system, said task management system comprising a plurality of staff profiles, said staff profiles comprising static and regularly updated dynamic properties of staff members, the method comprising:
- receiving a task request (100);
- retrieving a set of prioritization criteria (130) and respective associated ranking values and justification texts for said prioritization criteria;
- applying (140) a subset of said set of prioritization criteria to said plurality of staff profiles, and associating said associated ranking value and said associated justification text with each one of said staff profiles for each prioritization criterion of said subset that is fulfilled by said one of said staff profiles;
- selecting (150) for said task request a staff profile from among said plurality of staff profiles having the highest ranking value associated with it; and
- outputting (160) an assignment message comprising information indicative of said task request, information indicative of said staff profile selected for said task request, and said justification text associated with said highest ranking value;
wherein said subset of said set of prioritization criteria is determined so as to exclude prioritization criteria the negative outcome of which can be deduced by applying rules of formal logic to the outcome of other prioritization criteria within said subset.

2. The method according to claim 1, wherein said task request comprises at least one task attribute, and
wherein said retrieving of said set of prioritization criteria comprises associating prioritization criteria with said at least one task attribute.

3. The method according to claim 1 or claim 2, further comprising:
- retrieving a set of filter criteria (110) and respective associated ranking values and justification texts for said filter criteria;
- applying (120) a subset of said set of filter criteria to said plurality of staff profiles, and associating said associated ranking value and said associated justification text with each one of said staff profiles for each filter criterion of said subset that is fulfilled by said one of said staff profiles;
wherein said subset of filter criteria excludes filter criteria that are fulfilled by none of said staff profiles;
and wherein said subset of said set of prioritization criteria is only applied (120) to those of said plurality of staff profiles that fulfill all criteria of said subset of filter criteria.

4. The method according to claim 3, wherein said task request comprises at least one task attribute, and
wherein said retrieving (110) of said set of filter criteria comprises associating filter criteria with said at least one task attribute.

5. The method according to any of the preceding claims, wherein said applying is carried out by means of a rule-based reasoning engine.

6. A computer program product comprising code means configured to cause a processor to carry out the steps of any of the preceding claims.

7. A task request system (200) comprising a task management system (210) configured to carry out the method according to any of claims 1-5, a plurality of call terminals (220a-c), and a plurality of paging terminals (230a-c);
wherein said call terminals (220a-c) are operatively connected to said task management system (210) to convey a task request to said task management system (210); and
wherein said paging terminals (230a-c) are operatively connected to said task management system (210) to receive and display said assignment message.
